# EUROPÄISCHE PATENTSCHRIFT

(11) **EP 1 002 521 B1**
(45) Veröffentlichungstag und Bekanntmachung des Hinweises auf die Patenterteilung: **24.07.2002**
(21) Anmeldenummer: 98121938.9
(22) Anmeldetag: 19.11.1998
(51) Int. Cl.: A61K 7/32, A61K 7/48

(54) **Verwendung von Salbeiextrakten erhalten durch Extraktion mit Kohlendioxid als Deodorantien**
Use of sage extracts obtained by extraction with carbon dioxide as deodorants
Utilisation d'extraits de sauge obtenus par extraction au dioxyde de carbone comme deodorants

(43) Veröffentlichungstag der Anmeldung: 24.05.2000
(73) Patentinhaber: Heilmittelbetrieb Isernhagen GmbH, 61348 Bad Homburg (DE)
(72) Erfinder: Reinhard, Max, 61348 Bad Homburg (DE); Geissler, Jürgen, 01187 Dresden (DE)
(74) Vertreter: Schmidt, Horst, Dr.

(56) Entgegenhaltungen:
- WO-A-94/17814
- DE-A- 19 541 735
- FR-A- 2 345 143
- FR-A- 2 535 203
- FR-A- 2 753 374
- LU-A- 83 173
- "Verwendung von Herbasol-Extrakten in der Kosmetik" SEIFE-ÖLE-FETTE-WACHSE, Bd. 107, Nr. 20, Dezember 1981, Seiten 623-625, XP002096993 augsburg, DE
- STN, Karlsruhe, DE, Database Chemical XP002096994
- DATABASE WPI Week 8616 Derwent Publications Ltd., London, GB; AN 86-104817 XP002096995 & RO 88 082 A (NIVEA INTR PROD COS)
- DATABASE WPI Week 8929 Derwent Publications Ltd., London, GB; AN 89-209302 XP002096996 & JP 01 145063 A (LION CORP)

## Beschreibung

Die Erfindung betrifft die Verwendung von Extrakten aus Blüten und/oder Blättern von Salvia officinalis (Salbei) für Desodorierungszwecke. Ferner betrifft die Erfindung ein desodorierendes Präparat, das als Wirksubstanz einen Extrakt aus Blüten und/oder Blättern von Salbei enthält.

Aus "Lehrbuch der biologisches Heilmittel", Dr. Gerhard Madaus, (1938), S. 2406, ist es bekannt, dass Salvia officinalis (Salbei) ein gutes Regulationsmittel für Sekretionen, insbesondere für die Schweisssekretion, darstellt. Auch aus Römpp Chemielexikon, 9. Auflage (1993), S. 211 geht hervor, dass Salbei eine antihidrotische Wirkung, d.h. eine die Schweissentwicklung hemmende Wirkung, besitzt. Aus US-4 942 033 ist bekannt, dass ein Gemisch von Extrakten aus Mäusedorn (Ruscus aculeatus L.) und Salbei (Salvia officinalis L.) die Durchlässigkeit von Kapillaren verringert,was die Schweissabsonderung hemmen kann. Bekannt ist es demzufolge, die adstringierende Wirkung von Salbei auszunützen, um die schweisserzeugenden Poren möglichst weitgehend geschlossenzu halten und so das Auftreten von Körperschweiss zu verhindern. Keines der bekannten Präparate vermag jedoch die Schweissbildung vollständig zu unterdrücken, so dass eine Restschweissbildung nicht verhindert werden kann, die die Entstehung von unangenehmem Schweissgeruch zur Folge hat.

Körperschweiss an sich ist geruchlos, und erst eine bakterielle Zersetzung des Schweisssekerets führt zur Entwicklung von unangenehmen Gerüchen. Aus diesem Grund wurden desodorierende Präparate entwickelt, die eine antibakterielle Wirkung entfalten und somit eine Zersetzung des Schweisssekrets unter Geruchsentwicklung verhindern sollen. Ein Nachteil derartiger Präparate ist, dass sie relativ unspezifisch gegen eine Vielzahl an Bakterienarten wirken und somit auch die natürliche und erwünschte Bakterienflora der Haut beeinträchtigen können.

Aufgabe der Erfindung ist es, ein die genannten Nachteile bekannter Präparate gänzlich oder zumindest weitgehend ausschaltendes desodorierender Präparat zu schaffen. Dieses soll insbesondere eine spezifische Wirkung im wesentlichen nur gegen die für die Schweisszersetzung und somit Geruchsentwicklung verantwortlichen Bakterien ausüben.

Bei im Rahmen der Erfindung angestellten Untersuchungen an Salbeiextrakten wurde überraschend festgestellt, dass diese nicht nur eine antihidrotische Wirkung, sondern auch eine selektive bakterizide Wirkung gegen schweisszersetzende und damit für die Geruchsbildung verantwortliche Bakterien besitzen.

Die Erfindung sieht daher die Verwendung von Extrakten aus Blüten und/oder Blättern von Salvia officinalis (Salbei)für Desodorierungszwecke gemäß Anspruch 1 vor.

Ein weiterer Aspekt der Erfindung ist die Verwendung in einem desodorierenden Präparat, das als Wirkstoff einen Extrakt aus Blüten und/oder Blättern von Salvia officinalis zusammen mit üblichen Trägerstoffen und/oder Verdünnungsmitteln enthält. Dieses Präparat kann beispielsweise in Form von Sprays, Tinkturen, Pulvern und dergl. bereitgestellt werden. Eine besonders bevorzugte Darreichungform ist in Gestalt eines Deo-Stiftes oder sogenannten Deo-Rollers.

Eine besonders günstige Wirkung wurde bei Extrakten festgestellt, die unter Verwendung von überkritischem Kohlendioxid (CO₂) gewonnen wurden. Die überkritische CO₂ - Extraktion von Blüten und/oder Blättern von Salvia officinalis (Salbei) wird daher für die Erfindung bevorzugt da sie bei niedrigen Temperaturen durchgeführt werden kann und besonders schonend ist. Bezüglich weiterer Details in Verbindung mit der Herstellung von derartigen Extrakten kann beispielsweise auf WO/9417814 verwiesen werden.

Der Gehalt an Salbeiextrakt d.h. der Wirkstoffgehalt der desodorierenden Präparate beträgt vorzugsweise 0,5 bis 5%.

Die Extraktion der Blüten und/oder Blättern von Salvia officinalis kann jedoch auch mit Hilfe von beliebigen anderen Extraktionsmitteln, wie Wasser, organischen Lösungsmitteln etc. erfolgen. Ein Beispiel für ein organisches Lösungsmittel ist Ethanol. Die Temperatur bei der Extraktion und bei einer sich gegebenenfalls daran anschliessenden Stufe zur mindestens teilweisen Entfernung des Extraktionsmittels, z.B. durch Destillation, soll bei 50°C oder darunter und vorzugsweise bei 40°C oder darunter liegen, um eine thermische Beeinträchtigung der Inhaltsstoffe der Blüten und/oder Blätter zu verhindern. Dies bedeutet, dass bei der destillativen Trennung, der Druck soweit gesenkt werden muss, dass die genannte Temperaturobergrenze eingehalten werden kann.

Die Extraktion mit überkritischem CO₂ kann in beliebigen, dafür geeigneten Vorrichtungen erfolgen. Aus den thermodynamischen Eigenschaften von CO₂, nämlich einer kritischen Temperatur von 31,3°C und einem kritischen Druck von 71,5 bar, ergeben sich die unteren Grenzen für die Temperatur und für den Druck bei der Extraktion. Insbesondere wird es bei der CO₂-Extraktion bevorzugt, bei einer Temperatur von 40°C oder darunter zu arbeiten. Der Druck liegt vorzugsweise im Bereich von 90 bis 300 bar. Die Extraktion kann solange fortgesetzt werden, bis alle mit überkritischem CO₂ extrahierbaren Inhaltsstoffe aus den Blüten und Blättern von Salvia officinalis extrahiert worden sind. Dies ist üblicherweise nach einer Dauer des Extraktionsverfahrens von 1 bis 2 Stunden der Fall. Erfindungsgemäss ist es jedoch auch möglich, nur einen Teil der Inhaltsstoffe aus den Blüten und Blüttern von Salvia officinalis zu extrahieren.

Ein Vorteil der Verwendung von überkritischem CO₂ als Extraktionsmittel im Vergleich zur Verwendung anderer Extraktionsmittel, wie Ethanol oder Wasser, besteht darin, dass bei Temperaturen unter 40°C extrahiert werden kann, während z.B. bei einer herkömmlichen alkoholischen Extraktion das Abdestillieren des Ethanols bei Temperaturen von über 100°C erforderlich ist. Ein weiterer Vorteil der Extraktion mit überkritischem CO₂ besteht darin, dass lösungsmittelfreie Extrakte erhalten werden können. Eine ungünstige Beeinflussung der Wirkung des Extraktes durch Lösungsmittel, wie Ethanol, kann daher vermieden werden.

Die eingesetzten Blüten und Blätter von Salvia officinalis werden nach der Ernte vorzugsweise getrocknet, und zwar bei einer Temperatur von 40°C oder darunter. Die Trocknungstemperatur sollte wie die Extraktionstemperatur vergleichsweise niedrig gewählt werden um eine schonende Behandlung des Ernteertrags zuerhalten. Auch der Einsatz von tiefgefrorenem Salbei kommt grundsätzlich in Frage.

Der nach dem erfindungsgemässen Verfahren gewonnene Extrakt aus Blüten und/oder Blättern von Salvia officinalis liegt in pastenartiger Konsistenz vor. Dieser Extrakt kann als wirksamer Bestandteil eines desodorienden Präparats verwendet werden. Dieses kann in einer beliebigen Dareichungsform bereitgestellt werden. Dazu wird der Extrakt mit beliebigen geeigneten Verdünnungsmitteln, Füllstoffen oder dergl. versetzt und anschliessend in die gewünschte Dareichungsform überführt. Es bestehen keine speziellen Beschränkungen hinsichtlich des Verhältnisses von Extrakt zu Verdünnungsmittel, Füllstoff oder dergl. Die Herstellung des Präparats kann nach üblicher kosmetischer Praxis erfolgen.

Nachstehend wird die Erfindung an Hand von Beispielen näher erläutert.

### Beispiel 1

Es wird die Herstellung eines CO₂-Gesamtextraktes beschrieben. Dazu wurden handgepflückte, getrocknete Blüten von Salvia officinalis 2 Stunden bei einem Druck von 300 bar (vollständige Extraktion) und einer Temperatur von 40°C mit CO₂ extrahiert. Aus 15,2 kg Salbeiblüten wurden dabei 623 g Extrakt gewonnen. Dies entspricht einer Extraktausbeute von 4,1 %. Der Extrakt fiel als Paste an.

### Beispiel 2

In diesem Beispiel wird die Gewinnung eines CO₂-Selektivextraktes beschrieben. Die Extraktionsbedingungen umfassten eine Extraktionsszeit von 2 Stunden, einen Druck von 90 bar (selektive Extraktion) und eine Temperatur von 40°C. Aus 1,7 kg eingesetzten getrockneten Blüten von Salvia officinalis wurden dabei 14 g Extrakt gewonnen. Dies entspricht einer Extraktausbeute von 0,8 %. Der Extrakt fiel als Paste an.

### Beispiel 3

Es wurde wie bei Beispiel mit der Abänderung verfahren, dass Salbeiblätter anstelle von Salbeiblüten verwendet wurden. Man erhielt einen Gesamtextrakt in einer ähnlichen Ausbeute wie in Beispiel 1.

Die nachstehenden Beispiele 4 und 5 erläutern die Zusammensetzung von desodorierenden Präparaten, wobei die INCI-Nomenklatur für die Bestandteile verwendet wird.

### Beispiel 4

### Salbei-Deoroller-Emulsion:

Bestandteile: acqua, glyceryl stearate, cetearyl alcohol, paraffinum liquidum, Salvia officinalis, ceteareth-25, ceteareth-12, methyldibromoglutaronitrile, phenoxyethanol.

### Beispiel 5

### Salbei-Deoroller-Gel:

Bestandteile: acqua, alcohol denat., PEG-7 glyceryl cocoate polymer, Salvia officinalis, PEG-35, castor oil, hydroxyethylcellulose.

Das nachstehende Beispiel 6 beschreibt einen in-vitro-Test von Salbeiextrakten.

### Beispiel 6

Der Einfluss von Salbeiextrakten auf schweisszersetzende Bakterien wurde untersucht. Dazu wurden Verdünnungen der Präparate der Beispiele 1 bis 3 in einem Gemisch aus 72% Propylenglykol, 21% Wasser und 7% Natriumstearat hergestellt.

Die Versuche wurden wie folgt durchgeführt: Im Zentrum einer Nähragarplatte (in einer Petri-Schale) wurde eine Vertiefung ausgehoben. Eine mit der Testlösung getränkte Filterpapierscheibe wurde in die Vertiefung gelegt. Anschliessend wurde die Scheibe mit Nähragar bis zum Niveau der übrigen Agarfläche bedeckt. Nach Erstarren des Agar wurden die Platten mit Hilfe einer Öse mit verschiedenen Mikroorganismen inokuliert. Die Bakterienkolonien auf der Kontaktfläche des Nähragars mit der Papierscheibe wurden untersucht.

Das Bakterienwachstum wurde folgendermassen bewertet:
A: Angabe des Radius der Hemmzone vom Rand des beladenen Blättchens aus gemessen,
B: totale Inhibierung des Wachstums auf der Kontaktfläche,
C: minimales Wachstum (dünner Rasen) auf und am Rand der Kontaktfläche,
D: Wachstum auf der Kontaktfläche entsprechend der Negativkontrolle.

Die Bewertungsergebnisse sind in der nachstehenden Tabelle zusammengestellt. Es zeigt sich, dass beispielsweise eine 1%ige Salbeiblüten-Selektivextraktlösung eine starke wachstumshemmende Wirkung auf die für die Schweisszersetzung und damit für die Geruchsbildung verantwortlichen Keime, nämlich Staphylococcus aureus, Staphylococcus epidermidis und Corynebacterium spec., ausübt. Dagegen bleibt das Wachstum von zahlreichen anderen, zum Teil für die Haut typischen Keimen ungehemmt. Der Vorteil der erfindungsgemässen Präparate besteht somit unter anderem darin, dass selektiv schweisszersetzende Bakterien gehemmt werden, während die übrige Mikroorganismenflora grossenteils unbeeinträchtigt bleibt.

**Tabelle**

| **Extrakt von Beispiel** | **2** | **1** | **3** | **3** |
|---|---|---|---|---|
| **Konzentration** | **1%** | **1%** | **0,6%** | **1,0%** |
| **Keimart** | | | | |
| Staphylococcus aureus | A: 1mm | A:. 2mm | C | B |
| Staphylococcus epidermidis | A: 1-2mm | A: 2-3mm | C | B |
| Corynebacterium spec. | A: 6mm | A: 2-3mm | A: 3mm | A: 1 mm |
| | | | | |
| Escherichia coli | D | D | D | D |
| Proteus vulgaris | D | A: 1-2mm | D | |
| Klebsiella pneumoniae | D | D | D | D |
| Enterobacter aerogenes | D | D | D | D |
| Pseudomonas aeruginosa | D | D | D | D |
| Pseudomonas fluorescens | D | A: 1mm | D | D |
| Lactobacillus acidophilus | A: 2mm | A: 2mm | D | D |
| Peptostreptooccus prevotii | -* | - | D | B |
| | | | | |
| Candida albicans | D | D | D | D |
| Aspergillus niger | D | D | D | D |

| | | | | |
|---|---|---|---|---|
| *-: nicht bestimmt | | | | |

## Patentansprüche

1. Verwendung eines durch eine CO₂-Extraktion erhaltenen Extraktes aus Blüten und/oder Blättern von Salvia officinalis für die Herstellung eines desodorierenden Präparates.

2. Verwendung nach Anspruch 1, **dadurch gekennzeichnet, dass** der Extrakt in einem Desodorierungsstift, einem Spray oder einem Deo-Roller enthalten ist.

## Claims

1. Use of an extract obtained from flowers and/or leaves of Salvia officinalis by CO₂ extraction for producing a deodorising preparation.

2. Use according to claim 1, in which said extract is contained in a deodorant stick, a spray or a deso-roller.

## Revendications

1. Utilisation d'un extrait de fleurs et/ou feuilles de Salvia officinalis obtenu par extraction avec CO₂ pour la production d'une préparation désodorisante.

2. Utilisation selon la revendication 1, **caractérisée en ce que** l'extrait est contenu dans un bâton désodorisant, un spray ou un déo-roller.
